# EUROPEAN PATENT APPLICATION

(11) **EP 2 070 502 A1**
(43) Date of publication of application: **17.06.2009**
(21) Application number: 07816358.1
(22) Date of filing: 18.09.2007
(51) Int. Cl.: A61G 7/00, A47C 17/00

(54) **A SANITARY NURSING BED OF THERMAL INSULATION TYPE**

(30) Priority: 19.09.2006 CN 200620099098 U
(71) Applicant: Wang, Xiading, Fuzhou, Fujian 350004 (CN)
(72) Inventor: Wang, Xiading, Fuzhou, Fujian 350004 (CN)
(74) Representative: Hryszkiewicz, Danuta
(86) International application number: PCT/CN2007/002741
(87) International publication number: WO 2008/040166

(57) **Abstract**

A sanitary medical bed of thermal insulation type includes a frame (1) and supporting legs (2) and a sheet (3) with meshes is mounted on the upper surface of the frame (1), a thermal insulation bottom cover (4) is mounted on the lower surface of the frame (1), there is a space formed between the sheet (3) and the bottom cover (4), the space is communicated to an air conditioner (5).

## Description

The invention relates to a medical bed with a breathable and heat insulation function, and is suitable for bedridden paralyzed patients.

The existing medical beds are monotonous in function and each consists of a bedstead and a mattress disposed thereon. The mattress is generally made of heat insulation materials. In summer, a mat can be laid on the mattress for cooling. However, for a bedridden patient, due to poor breathability at the side of the body lying on the mat, the patient often suffers from bedsore or frostbite. In addition, since sickbed patients cannot take care of themselves, they often relieve the bowels and urine in bed, which makes it difficult to be thoroughly washed and leaves peculiar smell and further affects living environment of wards. A medical bed disclosed in my previous patent application suggests the use of a meshed mattress as a mattress so as to solve problems such as breathability and cleaning. However, a problem that the mesh leaks air and cannot facilitate heat insulation appears, which makes the invention unsuitable for use in autumn and winter. How to solve this problem becomes an object of the invention.

It is an objective of the present invention to provide a medical bed that is breathable and easy for cleaning and has a heat insulation function.

The invention is realized as follows: A medical heat insulation bed, comprising a bed frame and multiple supporting legs. A meshed body is disposed on an upper edge of the bed frame, and a heat insulation bottom cover is disposed on a lower edge of the bed frame so as to insulate heat at the bottom of the meshed body. An upper portion of a body is covered by a quilt. These two components clips the body so as to implement heat insulation and the meshed body is breathable and easy for cleaning.

The heat insulation bottom cover is disposed in a place where the hip of a patient is located and is in a shape of a concaved funnel, and a water outlet is disposed at the lowest position of the funnel and makes it possible for cleaning water to be discharged.

The heat insulation bottom cover at least comprises a strengthening layer, a heat insulation material layer and a waterproof layer disposed on the inner surface thereof. The strengthening layer ensures that the heat insulation material layer is fixedly attached and difficult to be damaged during cleaning. The waterproof layer affectively prevents the strengthening layer and the waterproof layer from being polluted by sewage or from erosion.

The invention is reasonable in design and simple in structure, and effectively solves a problem that a patient often suffers from bedsore or frostbite due to poor breathability caused by long-time lying on the bed. Moreover, it is convenient to clean the bed since sewage water can be directly discharged via a water outlet at the bottom thereof. If an air-conditioner is connected to the space formed by the meshed body and the heat insulation bottom cover, not only temperature can be freely adjusted, but also the air can be dehumidified, and thus the back of the patient can be kept dry and cool, which reduces the chance of suffering from skin diseases and further reduces difficulty in nursing the patent.

Detailed description will be given below in conjunction with accompanying drawings:

FIG. 1 is an exploded view of a medical heat insulation bed;

FIG. 2 is a perspective view of a medical heat insulation bed;

FIG. 3 is a schematic view of a medical heat insulation bed;

FIG. 4 is a cross-sectional view of a medical heat insulation bed;

FIG. 5 is a schematic view of an end portion of a medical heat insulation bed.

| | | |
|---|---|---|
| 1 - bed frame | 2 - supporting leg | 3 - meshed body |
| 4 - bottom cover | 5 - air conditioner | 11 - track |
| 12 - manipulator platform | 13 - motor | 40 - water outlet |
| 41 - funnel-shaped structure | 42- oil-feeding layer | 43 - heat insulation material layer |
| 44 - waterproof layer | 45 - through hole | |

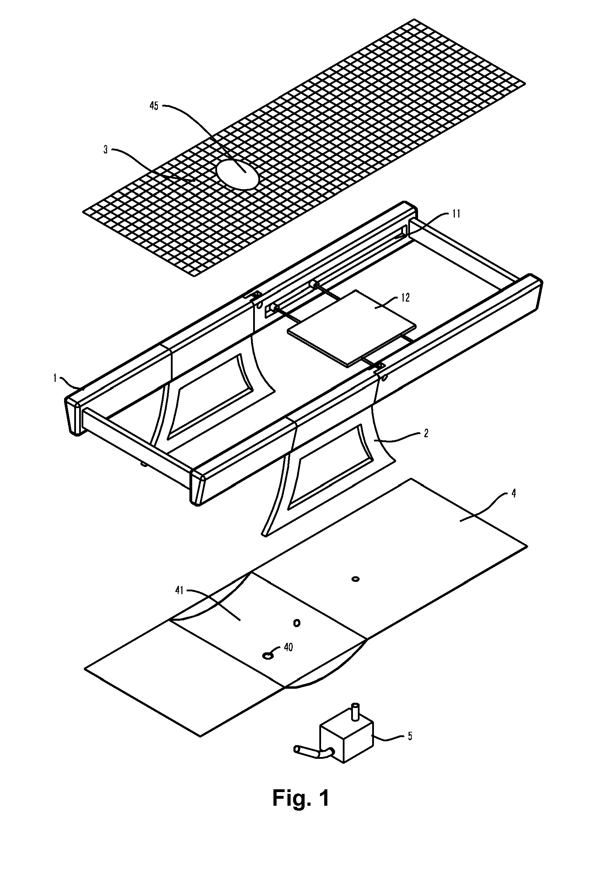

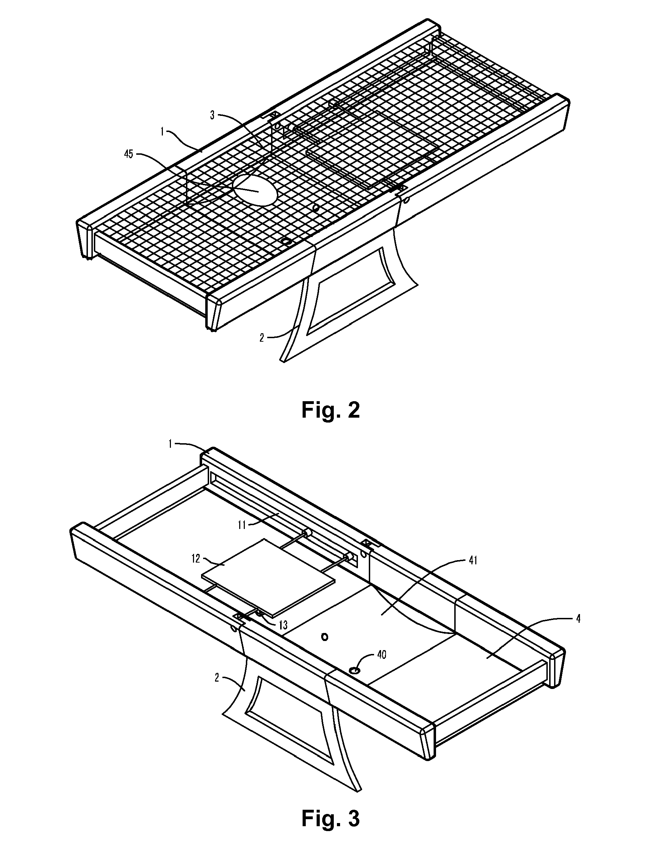

As shown in FIGS. 1, 2, 3, 4 and 5, a medical heat insulation bed of the invention comprises a bed frame 1 and multiple supporting legs 2. A meshed body 3 is disposed on an upper edge of the bed frame 1, and a heat insulation bottom cover 4 is disposed on a lower edge of the bed frame 1. There is space between the bottom cover 4 and the meshed body 3. This not only makes the air breathable, but also makes it possible to install other kinds of medical equipments. A place on the bottom cover 4 where a hip of a patient is located is a concaved funnel structure 41, and a water outlet 40 is disposed at the lowest position of the funnel so as to discharge sewage water from the meshed body.

Moreover, a spray header is disposed within space of the funnel-shaped structure 41, which makes it possible to spray upwards from the bottom of the meshed body 3 and to conduct cleaning from the upper side and the lower side without leaving dead space.

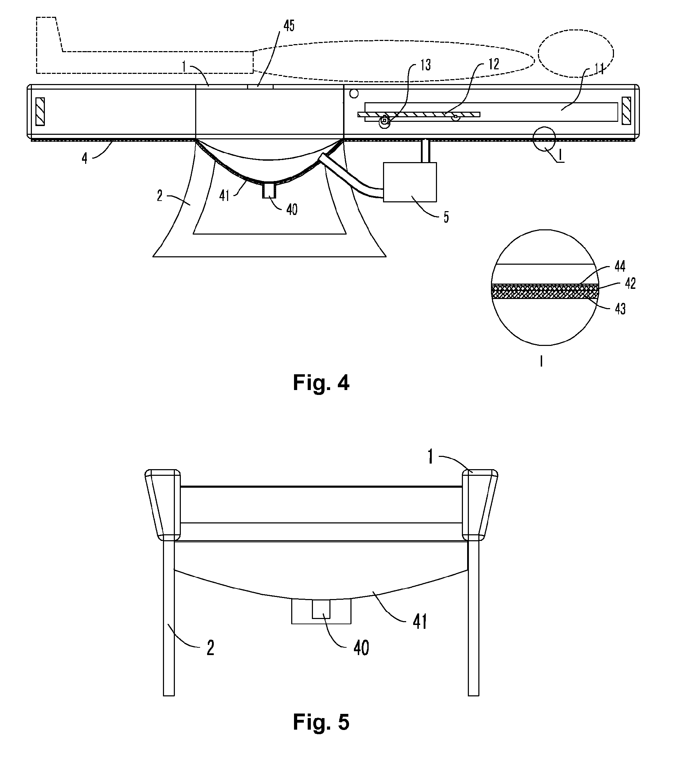

Referring to FIG. 4, the heat insulation bottom cover 4 at least comprises a strengthening layer 42, a heat insulation material layer 43 and a waterproof layer 44 disposed on the inner surface thereof. The strengthening layer 42 is made of stainless steel sheet, plastic or metal mesh materials and is corrosion-resistant. The heat insulation material layer 43 attached on the outer surface and the waterproof layer 44 attached on the inner surface effectively prevent sewage water from polluting the strengthening layer 42 and the heat insulation material layer 43. The waterproof layer employs a removable structure and is attached to the surface of the strengthening layer such as a stainless steel sheet, which enables it to be changed freely.

Referring to FIGS. 1, 3 and 4, a pair of corresponding tracks 11 are disposed on both sides of the bed frame 1, and a manipulator platform 12 is disposed between the tracks 11, The manipulator platform 12 is driven by an internal motor 13. The internal motor 13 drives the manipulator platform 12 to move up and down. The manipulator platform 12 makes it possible for various medial equipments, such as massage therapy equipments, infrared equipments and so on to be disposed thereon, so as to treat the back of patients. An air conditioner 5 is connected to the space formed by the meshed body 3 and the heat insulation bottom cover 4. The air conditioner adjusts environmental temperature and humidity in the bed frame to make them suitable for human body, which is beneficial for the health of the patients.

In addition, meshes of the meshed body 3 are sheet formed, and can be in the shape of a circular hole, a rectangular hole, a hexagon, an octagon and so on. A diameter or a maximum diagonal line of each mesh is less than 30 mm.

The meshed body 3 comprises multiple fiber lines interleaved with each other and may be interleaved to have multiple circular or geometrical-shaped meshes, and a diameter or a maximum diagonal line of the mesh is 30 times less than that of the fiber line.

The fiber line forming the meshed body 3 is made of transparent or semi-transparent materials, so that it is possible to observe inner space of the heat insulation bottom cover and to pass through spectrum such as infrared rays and ultraviolet rays. Materials forming the meshed body are as soft as possible.

In order to enable inconvenient patients to relieve bowels and urine, a through hole 45 is disposed on the meshed body where the hip of a patient is located. After the through hole is set, inconvenient patients are capable of directly relieving bowels and urine via the through hole, and thus nursing becomes convenient, the meshed body is not to be contaminated and task of cleaning the meshed body are greatly reduced.

## Claims

1. A medical heat insulation bed, comprising
a bed frame; and
multiple supporting legs;
wherein,
a meshed body is disposed on an upper edge of said bed frame; and
a heat insulation bottom cover is disposed on a lower edge of said bed frame.

2. The medical heat insulation bed of claim 1, wherein
said heat insulation bottom cover is disposed in a place where the hip of a patient is located and is in a shape of a concaved funnel; and
a water outlet is disposed at the lowest position of the funnel.

3. The medical heat insulation bed of claim 1, wherein a spray header is disposed within space of the funnel-shaped structure.

4. The medical heat insulation bed of claim 1 or 2, wherein said heat insulation bottom cover at least comprises a strengthening layer, a heat insulation material layer and a waterproof layer disposed on the inner surface thereof.

5. The medical heat insulation bed of claim 4, wherein said waterproof layer employs a removable structure and is attached to the surface of said strengthening layer.

6. The medical heat insulation bed of claim 1, wherein
a pair of tracks are disposed on both inner sides of said bed frame;
a manipulator platform is disposed between said tracks; and
said manipulator platform is driven by an internal motor.

7. The medical heat insulation bed of claim 1 or 2, wherein an air conditioner is connected to space formed by said meshed body and said heat insulation bottom cover.

8. The medical heat insulation bed of claim 1, wherein a diameter or a maximum diagonal line of each mesh of said meshed body is less than 30 mm.

9. The medical heat insulation bed of claim 1, wherein
said meshed body comprises multiple fiber lines interleaved with each other;
said fiber line is made of transparent or semi-transparent materials; and
a diameter or a maximum diagonal line of the mesh is 30 times less than that of said fiber line.

10. The medical heat insulation bed of claim 1, 2 or 3, wherein a through hole is disposed on said meshed body where the hip of a patient is located for defecating.
